# EUROPEAN PATENT APPLICATION

(11) **EP 4 300 511 A1**
(43) Date of publication of application: **03.01.2024**
(21) Application number: 22182056.6
(22) Date of filing: 29.06.2022
(51) Int. Cl.: G16H 50/20, G16H 10/60, G16H 40/20, G06F 40/10, G06F 40/174, G06N 3/04

(54) **CLINICAL TRIAGE BASED ON TEXT CLASSIFICATION**

(30) Priority: 28.06.2022 PT 2022118072
(71) Applicant: Universidade de Évora, 7000-803 Évora (PT)
(72) Inventor: De Freitas Gonçalves, Teresa Cristina, 2805-290 ALMADA (PT); Vieira, Renata, 7000-677 ÉVORA (PT); Veledas, Rute, 9700-614 VILA DE SÃO SEBASTIÃO (PT); Yang, Hua, 7000-898 ÉVORA (PT); Quaresma, Paulo, 7004-516 ÉVORA (PT)
(74) Representative: Couto, Cláudia

(57) **Abstract**

The present application describes an automatic text classification method to aid on clinical referral of patients. The herein disclosed text classification method to aid on clinical referral of patients based on the selection of a clinical pathway comprises collecting personal and clinical information about a patient; inserting the personal and clinical information in text format in an application form; obtaining through the application form a text result based on the inserted personal and clinical information that identifies the clinical pathway for the contact reason of the patient.

## Description

### Technical Field

The present application describes an automatic text classification method to aid on clinical referral of patients.

### Background art

According to the EuroHealth Consumer Index, seventeen European countries had a form of telephone-based clinical triage in 2018. The triage procedure is based on the selection of a clinical algorithm considering the citizen's self-reported symptoms and signs, as well as relevant information from their medical history. The choice of the most appropriate clinical pathway is extremely important and relevant, as it should ensure high security (while not failing to identify situations that require urgent medical contact) and have high discriminatory capacity (not sending situations of low clinical risk to the emergency of hospitals).

This is a rather complex problem because there are usually a large number of clinical algorithms, which, for example, in Portugal, in the National Health Service, had before COVID pandemic, 56 different clinical algorithms.

Following the pre-defined clinical pathways, the health professional or nurse will select the most appropriate one according to the citizen's self-reported symptoms and relevant information about medical history. The selection of the clinical pathway leads to five possible final referrals: self-care, clinical assessment at a primary health care center, clinical assessment in hospital emergency, transference to the National Medical Emergency Institute, or transference to the Poison Information Center. All the existing clinical pathways start by asking screening questions, aiming to identify critical situations which need immediate health care and that are rapidly transferred to National Medical Emergency Institute. For non-emergencies, nurses must ensure that the main symptoms reported by the citizen are correctly mapped to allow the choice of the clinical pathway. Age, gender, and clinical history should also be considered during this process since this information is often crucial to the selection of the most appropriate clinical pathway. The choice of the clinical pathway by the health care professionals in each telephone triage episode is extremely important since it will determine the final referral. These pathways use a riskaverse system of prioritization, as other triage protocols such as the Manchester Triage System. For this reason, the system has an important role in the identification of users' clinical situation and the correct referral to health services according to their symptoms.

The use of clinical decision support systems improves therefore the quality of telephone triage service and care performance. If its recommendations are followed, more appropriate ER referrals are obtained, achieving cost reduction and effectiveness in patient forwarding to the most appropriate clinical speciality. Moreover, this will allow a better and faster interaction with the user, leading to a decrease service time and, consequently an increase the line capacity.

### Summary

The present invention describes a text classification method to aid on clinical referral of patients based on the selection of a clinical pathway, said method comprising collecting personal and clinical information about a patient; inserting the personal and clinical information in text format in an application form; obtaining through the application form a text result based on the inserted personal and clinical information that identifies the clinical pathway trial classification of the patient.

In a proposed embodiment of present invention, the application form is instanced through a web application service supported by a database.

Yet in another proposed embodiment of present invention, the web application service comprises at least one of three deep learning based architecture models, particularly a convolutional neural network or a recurrent neural network or transformers-based architecture.

Yet in another proposed embodiment of present invention, the web application service comprises the steps of recording the personal and clinical information of a patient; pre-processing the personal and clinical information; applying the personal and clinical information to the at least one of three deep learning models; obtaining a prediction most probable clinical pathway.

Yet in another proposed embodiment of present invention, the web application service comprises an input layer where the personal and clinical information of a patient comprising contact reason and clinical pathway will be inserted; at least one hidden layer composed at least of embeddings and pre-trained word embeddings or language models; and an output layer composed of the prediction.

Yet in another proposed embodiment of present invention, the web application service comprises the steps of Fine-Tuning of the machine learning model.

### General Description

The present application describes an automatic text classification method to aid on clinical referral of patients.

Health assistance centres play an important role in the identification of users' clinical situations according to their symptoms. Currently, there are a number of possible clinical pathways defined, and selecting the appropriate one is very important in each telephone triage episode. Decreasing the duration of the phone calls and allowing a faster interaction between citizens and said service can further improve the performance of the telephone triage service.

The herein disclosed solution describes therefore a new procedure to support a health assistance center for triage of users with health complaints. The triage of said patients is based on a given description (in Natural Language) provided by phone call to the health assistance center, which will disclose a set of the most suitable clinical pathway for the identified symptoms.

Currently the health assistance telephone line service is conducted by nurses. After an initial triage about the emergency of the call, the nurse collects personal information about the user and the reason(s) that led him/her to contact the health line. Based on the user's health history and the information collected, the nurse selects one of existing clinical pathways. It is then based on this pathway that the user is counselled and clinically referred.

This new procedure aims to assist the previously mentioned nurses in the process of selecting the most suitable clinical pathway for the patient. The procedure is instanced through a web service that, based on the written text describing the reason for the users' contact, provides the nurse with the list of the most probable clinical pathways (ordered by their probability).

For example, if when speaking with the patient, the nurse enters as the reason for contact, lets say "Fever + dry cough + rhinorrhoea in a 19 weeks pregnant woman for 24 hours" the herein disclosed system will indicate as a result "Cough" as the most likely pathway (56.08%) followed by "Flu Syndrome" (37.32%).

Although the procedure has been instantiated and initially developed to support the health assistance hotlines, it can be replicated for other hotlines for clinical triage.

The developed technical solution provides advantages on:
* Support in deciding the most appropriate clinical pathway to the patient condition;
* increased quality and faster interaction with the patient;
* Decrease of call time in attendance;
* Increase in the line's attendance capacity;

Since Machine Learning and Natural Language Processing techniques have been increasingly used in clinical decision support systems, the effort to apply them to clinical narratives aim to improve the overall performance of the developed systems with the use of newer techniques such as Support Vector Machines and Deep Learning architectures. Within one of the adopted solutions, the use of deep learning approaches is also one of the solutions to build classification models, aiming to support the nurses with the clinical pathway's choice.

This developed system explores the use of neural network (NN) architectures to build classification models for a health assistance telephone line service clinical triage, aiming to improve their quality and performance; support the nurses in the triage process, particularly with clinical algorithm's choice; decrease the duration of the phone calls and allow a finer and faster interaction between citizens and phone delivery service.

The main developments disclosed within the current invention are summarized as follows:
- Three deep learning (DL) based classification architectures are compared, namely CNN (convolutional neural network), RNN (recurrent neural network), and transformers-based architecture, for the automatic clinical pathway selection for patient health calls;
- An empirical process for fine-tuning hyperparameters is explored which shortens the tuning time and theoretically achieves near-optimal classification accuracy;
- Obtained comprehensive experimental results illustrate that the used neural network models outperform shallow machine learning models with the testing accuracies on the same clinical dataset;
- Useful suggestions are obtained and then provided to the health call center, with practical analysis among similar clinical symptoms. Predictions from triage, neural network models, and shallow machine learning models are compared.

Three different deep learning architectures, namely convolutional neural network (CNN), recurrent neural network (RNN), and transformers-based approaches are applied across a total number of nearly 270000 training records belonging to 51 classes, and an additional selection of the best combination of ten text representations and four machine learning algorithm was pursued by building 40 different models. The CNN, RNN, and transformers-based model allow to obtain an accuracy of 76.56%, 75.88%, and 78.15% over the test set in the preliminary experiments. Training models using the transformers-based architecture are further fine-tuned, achieving an accuracy of 79.67% with Adam and 79.72% with SGD after learning rate fine-tuning; an accuracy of 79.96% with Adam and 79.76% with SGD after epochs fine-tuning; an accuracy of 80.57% with Adam after the batch size fine-tuning. Analysis of similar clinical symptoms is carried out using the fine-tuned neural network model. Comparisons are done over the labels predicted by the neural network model, the support vector machines model, and the original labels. These results suggest that using deep learning is an effective and promising approach to aid the clinical triage of the phone call services.

The fine-tuning of the Support Vector Machine algorithm parameters and the text embedding model were performed achieving a final accuracy of 78.80% and F1 of 78.45%. The best setup was then used to calculate the accuracy of the top-3 and top-5 most probable clinical pathways, reaching values of 94.10% and 96.82%, respectively. These results suggest that using a machine learning approach to aid the clinical triage in phone call services is effective and promising.

In the developed system, a form receives the medical data describing the reason of the user for contact, and additionally the number of algorithms to be presented, and returns the most probable clinical algorithms. The interface has a "Clear Results" option to facilitate viewing of new examples whenever necessary.

### Brief description of the drawings

For better understanding of the present application, figures representing preferred embodiments are herein attached which, however, are not intended to limit the technique disclosed herein.
Figure 1 depicts the trained neural network model used to predict the clinical pathway given the information collected by the nurses. It illustrates the application of deep learning approaches on the clinical trial classification. The clinical texts which are recorded by the nurses are used as the input of the neural network models. Using the designed deep learning architectures, neural network models are trained to predict clinical pathways and provide suggestions to the nurses.

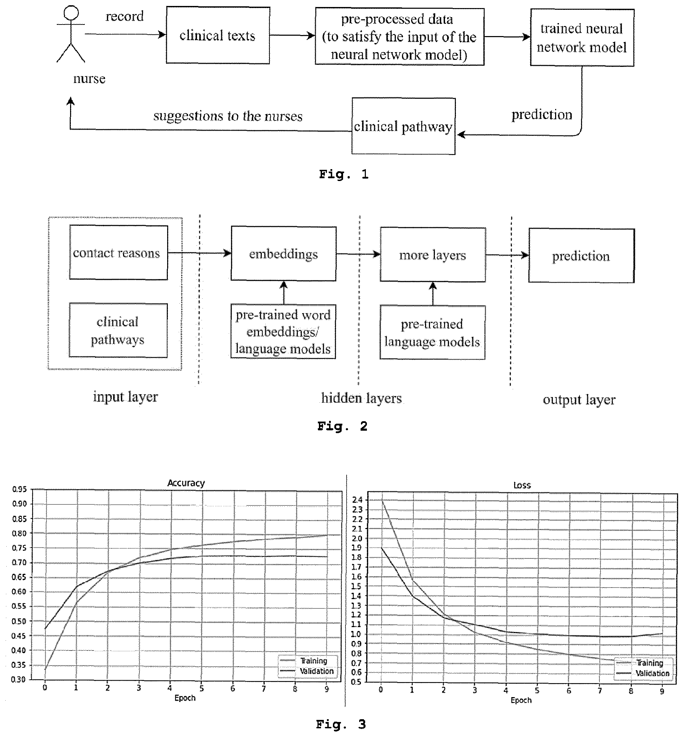

Figure 2 illustrates the general framework for training the neural network model of the disclosed invention. The design of a neural network model is highly depending on the selected architecture, such as the number of layers, the design of hidden layers, the pre-trained language model selection, etc. The training of the neural network models using CNN, RNN, and transformers-based architectures comprises Classical CNN-based architecture for text classification; RNN-based architecture with gated recurrent unit (GRU); Sequence-to-sequence transformers-based architecture for natural language processing tasks. The input layer, hidden layers, and output layer are designed for each of the three neural network architectures accordingly. In the input layer, the "contact reason" and "clinical pathway" are fed to the neural network. In the hidden layers, the pre-trained word embeddings or language models are selected for each architecture accordingly. The output layer is designed to do the predictions. For each architecture, "contact reason" and "clinical pathway" are used as the examples and labels. They are fed to the neural network as the input. When embedding the input "contact reason" texts pre-trained language models are used depending on the neural network architecture selection.
Figure 3 illustrates the training and validation results using the CNN-based architecture. (Left) : Accuracy vs. epochs; (Right) : Loss vs. epochs.
Figure 4 illustrates the rraining and validation results using the RNN-based architecture. (Left): Accuracy vs. epochs; (Right): Loss vs. epochs.
Figure 5 illustrates the training and validation results using the transformers-based architecture. (Left): Accuracy vs. epochs; (Right): Loss vs. epochs.
Figure 6 illustrates the Performance of the improved transformers-base model. This model uses the Adam optimizer and a learning rate of 1×10⁻⁶ over 150 epochs. The other hyperparameters are set the same as the model built in the preliminary experiments.
Figure 7 illustrates the performance of the improved transformers-base model. This model uses an SGD optimizer and a learning rate of 1×10⁻⁴ over 150 epochs. The other hyperparameters are set the same as the model built in the preliminary experiments.
Figure 8 illustrates the validation accuracy comparison between SGD and Adam over 150 epochs using transformers-based architecture.
Figure 9 illustrates the analysis among clinical pathways with similar clinical symptoms using the trained neural network model. This group includes six clinical pathways: "Pr. da orofaringe" (Oropharynx problem), "Pr. deasma ou pieira" (Asthma or wheezing problem), "Pr. Nasal" (Nasal problem), "Pr. Respiratório" (Respiratory problem), "Sindrome gripal" (Flu syndrome), "Tosse" (Cough).
Figure 10 illustrates the analysis among clinical pathways with similar clinical symptoms using the trained neural network model. This group includes three clinical pathways: "Diarreia" (Diarrhea), "Dor abdominal" (Abdominal pain), and "Náuseas e vômitos" (Nausea and vomiting).

### Description of Embodiments

With reference to the figures, some embodiments are now described in more detail, which are however not intended to limit the scope of the present application.

The original dataset used for the training of the previously mentioned deep learning architectures was provided from real life interactions within a 24h phone-line service. It contained information of call records received within a total of 270000 records with 18 fields.

Table 1 lists all 18 fields both in the original Portuguese and in English languages terminology. Each call record includes personal data, such as age, gender, and encrypted primary care unit. It also contains the calling information, such as the start/end time, initial intention, comments, contact reason, clinical pathway, and final disposition. The "contact reason" and "comments" fields are free text written in Portuguese by the receiving technician or nurse who answered the respective call. The date related information is recorded in date format and the other remaining fields are nominal attributes.

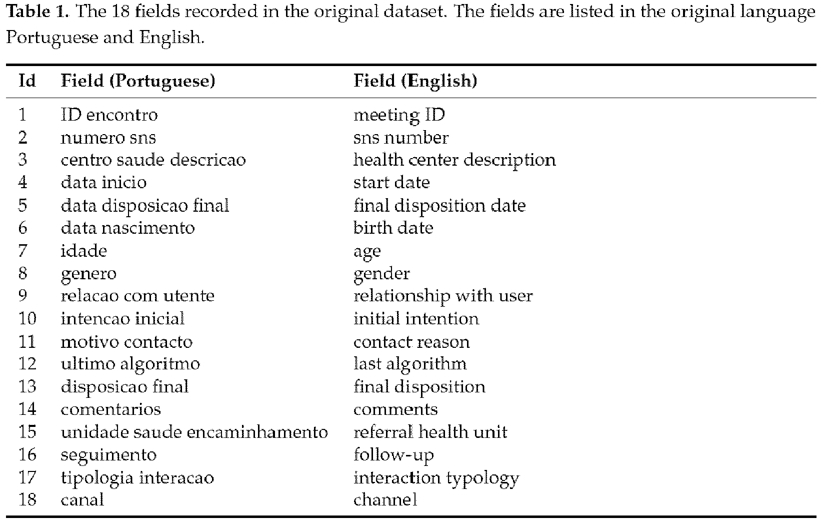

Within the original training dataset, there are 52 clinical pathways from the total of 59 defined by the SPMS, the proportion for each clinical pathway varying significantly. As an example, the term "Tosse" (Cough) has the highest number of records and accounts for 14.006% of the calls, while "Pr. por calor" (Heat problems) represents only 0.001% of the calls; six of the clinical pathways have over 10,000 calls, and three under 100. Table A1, lists the existing 52 clinical pathways, their record numbers, and the proportions to the whole dataset. The "contact reason" field was selected as a discriminant information field and is defined by a medium-length free text, consisting of straightforward information about the patient's problem.

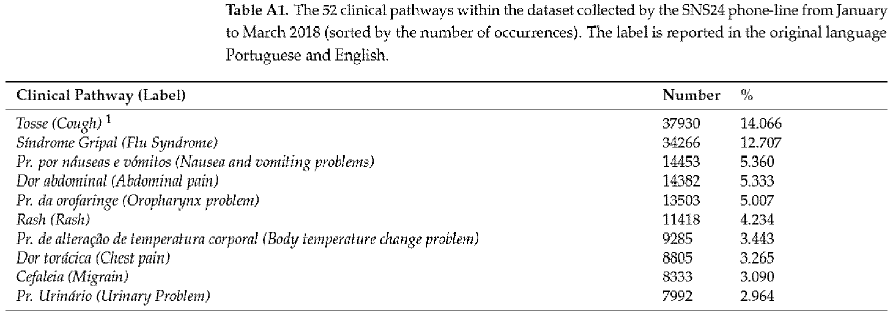

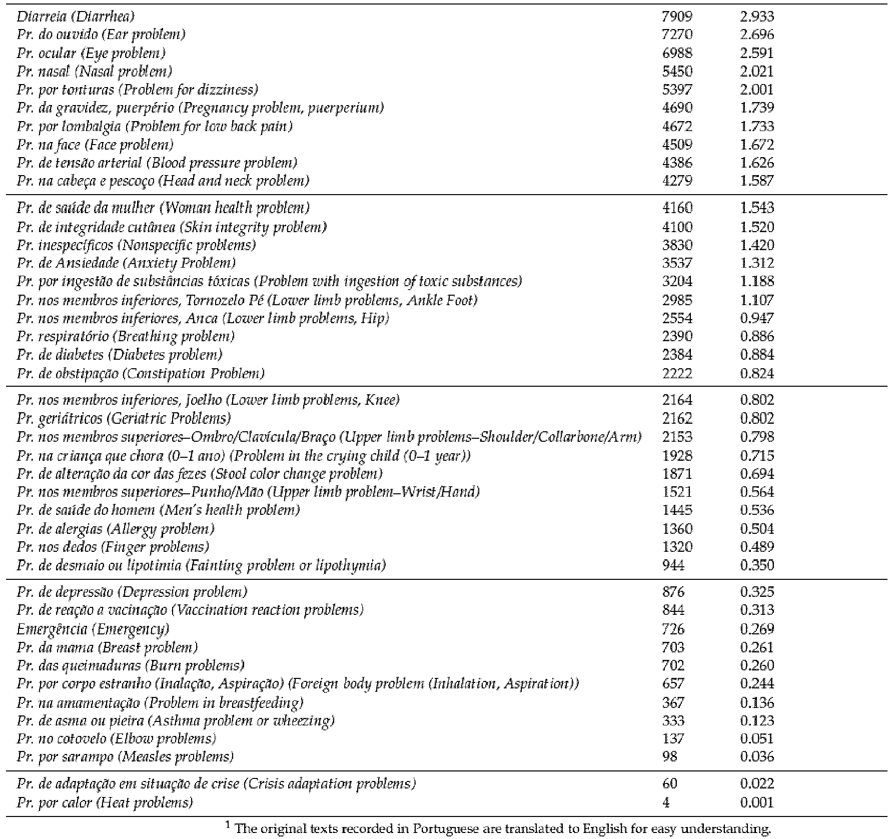

Table A2 discloses examples of "contact reason" field texts from the five most frequent clinical pathways and their respective label.

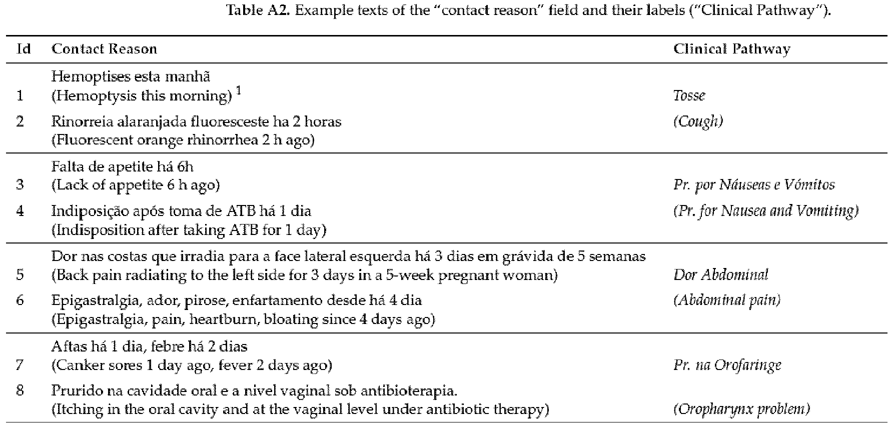

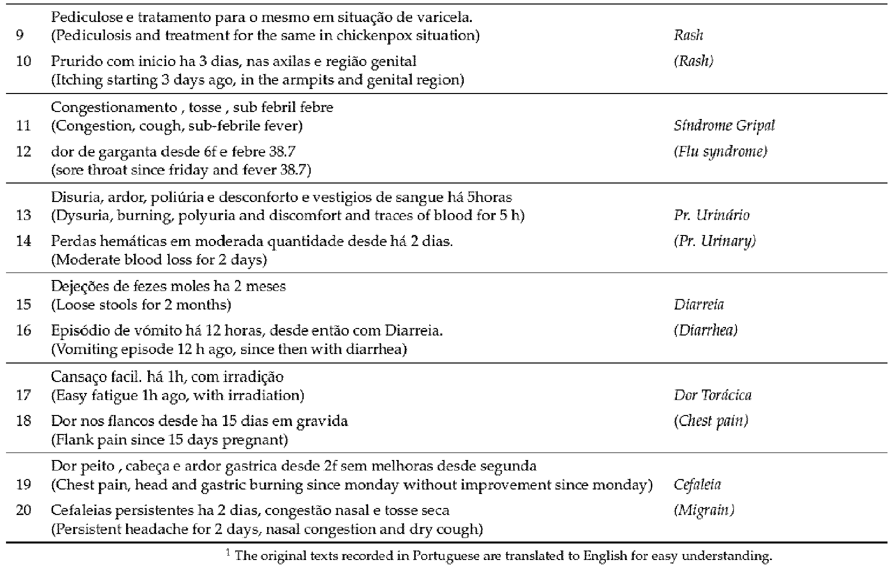

As a summary, the statistics of the original and experimental dataset are presented in Table 2.

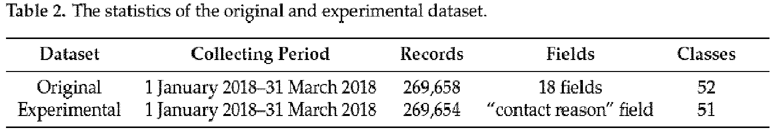

### Experimental setup

As the first step, we preliminarily experiment with all three neural network architectures over a small number of epochs. Based on the results obtained from the preliminary experiments, the following step is to select the best-performed architecture among all three and further fine-tuning the models with selected hyperparameters step by step. The final best fine-tuned model will be used for class prediction.

Therefore, the use of a shallow machine learning approach enables to predict the clinical pathways on the dataset. Different from deep learning approaches, shallow machine learning approaches or conventional machine learning approaches usually need human intervention in feature extraction. Typical shallow machine learning approaches include Linear/Logistic Regression, Decision Tree, SVM, Naive Bayes, Random Forest, etc. It is also used the same train/validation/test splits of the dataset. The dataset is stratified split into the train (64%), validation (16%), and test set (20%). The neural network models are trained over the train set and adjusted over the validation set, and the test set is used for the final evaluation of the models. The original dataset contains 18 fields (attributes), and only the texts of the "contact reason" field and the labels of the "Clinical pathway" field are used in building a prediction model. The texts from the other fields of the dataset are not used. Example texts and labels are shown in Table A2.

### CNN-Based Architecture

The used CNN architecture on the present invention comprises four layers: embedding layer (input layer), convolutional layer, pooling layer, and fully connected layer. When applying the CNNs in text analysis, the input has a static size and text lengths can vary greatly, so, as the input, the texts from the "contact reason" field are tokenized, and all words are integer encoded. These pre-processed train data are then fed to the CNN architecture. In the embedding layer, each word is embedded into a 200-dimension vector. A one-dimensional convolution layer is then added. Filters perform convolutions on the text matrix and generate feature maps. One depicts a filter region size. The max-pooling is performed over each feature map to select the most prominent feature (the largest number), then a max-pooling is chosen as the pooling strategy since it presents much better performance compared to average pooling in the text classification tasks. The max-pooling results of word embeddings are concatenated together to form a single feature vector. To prevent the built CNN model from overfitting, a dropout layer is added after the fully connected layer. The final Softmax layer receives the concatenated feature vector as input and uses it to classify the texts. The main hyperparameters settings in the built CNN model are shown in the second column of Table 3.

The training and validation results are presented in Figure 3. The left figure shows the accuracy's on the train and validation datasets. As it can be observed, the accuracy increases slowly on the training dataset after 6 epochs. The accuracy on the validation dataset tends to saturate after certain epochs, and the curve doesn't show the trend of increasing accuracy after more epochs within the setting one. Figure 3 right plots the loss curves of the train and validation datasets over the setting epochs.

**Table 3. Hyperparameter settings in the neural network models for different architectures. The first column lists the main hyperparameters used in all three neural network architectures, and the hyperparameters that are not used or not manually set in our experiments in one architecture are marked with "-" in the table. CCEE stands for categorical cross entropy error.**

| Hyperparameters | CNN-Based | RNN-Based | Trans formers-Based |
|---|---|---|---|
| Hidden layers | 4 | 3 | 12 |
| Hidden size | - | 512 | 768 |
| Filter size | 3 | - | - |
| Feature map | 128 | - | - |
| Pooling strategy | 1-max pooling | - | - |
| Activation function | ReLU, Softmax | - | Gelu [31] |
| Regularization strategy | Dropout | Dropout | Dropout |
| Dropout rate | 0.1 | 0.1 | 0.1 |
| Batch size | 128 | 128 | 128 |
| Patience | - | 5 | - |
| Anneal factor | - | 0.5 | - |
| Optimizer | Adam | Adam | Adam |
| Learning rate | 1 × 10⁻⁴ | 1 × 10⁻⁴ | 1 × 10⁻⁴ |
| Loss function | CCEE | CCEE | CCEE |
| Epoch | 10 | 10 | 10 |

The loss curve on the training dataset decreases rapidly at the beginning of 3 epochs and slowly after the 3 epochs. The loss curve on the validation dataset saturates gradually after 6 epochs, and the loss has been reduced to a minimum level at epoch 8 and then begins to increase. As observed from both figures, the curves have been basically stable after 6 epochs within the setting epochs. Using this trained CNN model, the accuracy obtained on the test dataset is 76.56%.

### RNN-Based Architecture

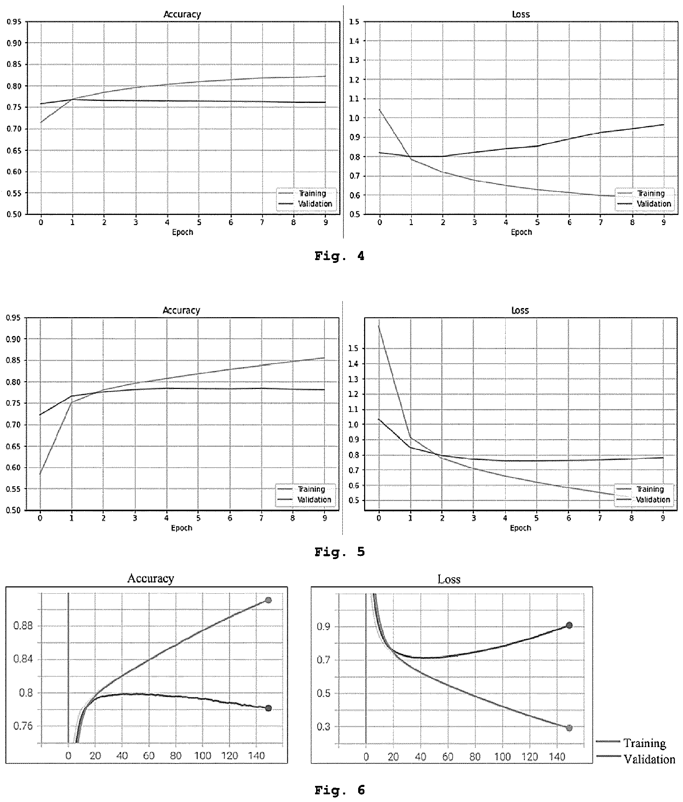

When constructing the RNN-based neural network, a pre-trained Flair model is chosen for the implementation. Flair is a natural language processing(NLP) library and builds on PyTorch which is one deep learning framework. A group of pre-trained models for the NLP tasks is provided in Flair. We choose the pre-trained Flair model that provides text classification ability as part of the implementation of our RNN-based model. The pre-trained text classification model takes word embeddings, puts them into a recurrent neural network (RNN) to obtain a text representation, and puts the text representation in the end into a linear layer to get the actual class label [28]. The list of the word embeddings from one text is passed to the RNN, and the final state of the RNN is used as the representation for the whole text. The GRU-type RNN is used in our experiments. The main hyperparameters settings used are shown in Table 3. Figure 4 plots the accuracy and loss observed on the training and validation dataset using the RNN-based architecture. From the results, we can see that this model overfits too early after only a few epochs. The best score obtained on the testing dataset is 75.88%.

### Transformers-Based Architecture

To obtain the transformers-based neural network for our task, we chose to use the pre-trained BERT model as the implementation of transformers. We choose a pre-trained BERT model that provides text classification ability. With TensorFlow, we first instantiate this classification model with a pre-trained model's configuration from a BERT Portuguese model; then, we fit the model to our dataset. We use BERTimbau Base, which is a pre-trained BERT model for Portuguese that achieves state-of-the-art performance on a number of NLP tasks. In particular, we use the base model of BERTimbau Base, which includes 12 layers. The pre-processed data is then fed to the neural network. The main hyperparameter values used in the transformed-based architecture are summarized in the fourth column of Table 3. Figure 5 depicts the accuracy and loss curves on the training and validation dataset. The loss on the validation dataset has been reduced to a minimum level at epoch 5. Using the transformers-based architecture, the accuracy score obtained on the testing dataset is 78.15%.

### Fine-Tuning of the Transformers-Based Models

Default or empirical hyperparameter settings are used upon building the prediction model. To adapt the neural network models, a fine-tune the hyperparameter is set to the target dataset. Since the transformers-based model presents better performance than the CNN and RNN models in the preliminary experiments, further fine-tune is performed over the hyperparameters of the transformers-based model, aiming to achieve better results.

### Fine-Tuning Strategy

Our fine-tuning aim is: (1) an ideal optimizer with an appropriate learning rate; (2) an ideal batch size for training on the target dataset. So, we mainly consider three hyperparameters, learning rate, optimizer, and batch size. Since it is costly to train transformers-based models, the fine-tuning process is designed as the following:
- Fine-tuning of learning rate (abbr. lr). During the first phase, we experiment with small epochs (e.g., 10) and range with different learning rates on the different optimizers.
- Fine-tuning on epochs. Based on the results observed from the first phase, we analyse the accuracy and loss curves to choose the best learning rate for each optimizer. We then use that group of hyperparameters and set the epoch to a large number for further training.
- Fine-tuning on batch size. Based on the results obtained after fine-tuning on learning rates and epochs, the final fine-tuning is done by adjusting the batch size with a set of varying values. In such a way, we can better save the resources in training the models and shorten the time in fine-tuning. This method practically eliminates the need to repeatedly tune on large epochs every time and theoretically achieves near-optimal classification accuracy. We will describe each phase strategy in more detail in the following subsections.

### Optimization Algorithm Choosing

The optimization algorithm (a.k.a. optimizer) is one main approach used to minimize the error when training neural network models. A number of optimizers have been researched and generally used ones include Stochastic Gradient Descent (SGD), Momentum Based Gradient Descent, Mini-Batch Gradient Descent, Nesterov Accelerated Gradient (NAG), Adaptive Gradient Algorithm (AdaGrad), Adaptive Moment Estimation (Adam), etc.. When choosing an optimizer for fine-tuning the neural network models, the speed of convergence and the generalization performance on the new data are usually considered.

In our work, we mainly take into account two widely used optimizers in our experiments, Adaptive Moment Estimation (Adam) and Stochastic Gradient Descent (SGD) since these two optimizers present better performances than the others in many NLP tasks. When training neural network models, Adam is one of the most practical optimizer. Adam is an algorithm for gradient-based optimization and combines the advantages of two SGD extensions: RMSProp and Adagrad. Adam can compute adaptive learning rates for different parameters individually. As a variant of Gradient Descent (GD), SGD is a computationally efficient optimization method on large-scale datasets. When doing experiments on a large-scale dataset, computations over the whole dataset are usually redundant and ineffective. SGD can do computations on a small or a randomly selected subset instead of the whole dataset.

### Fine-Tuning of Learning Rate

During this phase, we explore the effect of different learning rate settings. In particular, we focus on the variants of the learning rate with each optimizer. The setting of the learning rate plays a decisive role in the convergence of a neural network model. A too-small learning rate will make a training algorithm converge slowly, while a too-large learning rate will make the training algorithm diverge. A traditional default value for the learning rate is 0.1, and we use this as a starting point on our task problem. For each optimizer (Adam and SGD), we try learning rates within the set of {0.1, 0.01, 1×10⁻³, 1×10⁻⁴, 1×10⁻⁵, 1×10⁻⁶, 1×10⁻⁷}. We maintain the same training procedure as the preliminary models described in Section 4.2.3 except for changing the learning rate. We use the same number of epochs (epochs = 10) as a start. The rest of the hyperparameters remain the same.

When varying the learning rate settings with optimizer Adam (Figure A1) and SGD (Figure A2), the training and validation curves of the train and validation dataset are depicted. The results on accuracy as well as loss values are reported. Observing the curves learned by using Adam with different learning rates, we find that when setting the learning rate as 1×10⁻³ or bigger, the training models fails to converge. Especially when the learning rate is set with an aggressive value of 0.1, we observe that the loss increases rather than decreases. When the value is set as 1×10⁻⁴ or 1×10⁻⁵, the two models archive similar performance, and the models converge too early on the validation dataset. We also observe that the loss on the validation dataset increases with more epochs. When setting a rather small value of 1×10⁻⁷, we observe that the loss decreases too slow compared to the other learning rates (the loss is around 2.3 after 10 epochs). When setting the learning rate as 1×10⁻⁶, we observe that the results clearly show a downward trend in loss and upward in accuracy over the epochs. This sign shows the model is learning the problem and has learned the predictive skill.

So, when choosing Adam as the optimizer, the appropriate setting of learning rate is 1×10⁻⁶. The loss curves of the training and validation datasets are decreasing, and they are still showing downward trends by the pre-defined epochs. This observation suggests that the prediction performance could be potentially improved with more epochs (training with more epochs than 10). The best accuracy on the test dataset achieves a score of 79.67%, obtained with the learning rate of 1×10⁻⁵ and Adam optimizer. When varying the learning rate setting with optimizer SGD, we can analyse the results similarly as with the Adam optimizer. We observe that the model fails to converge when the learning rate is set as 0.1 or 0.01; the model converges too early with a learning rate of 1×10⁻³; the loss decreases very slow when the learning rate is 1×10⁻⁵ or smaller. The best accuracy on the test dataset achieves a score of 79.72%, obtained with the learning rate of 1×10⁻³ and SGD optimizer. But the curve shows no obvious downward trend on 1×10⁻³ after 10 epochs. So, with SGD as the optimizer, the appropriate learning rate setting is 1×10⁻⁴.

### Fine-Tuning on Epochs

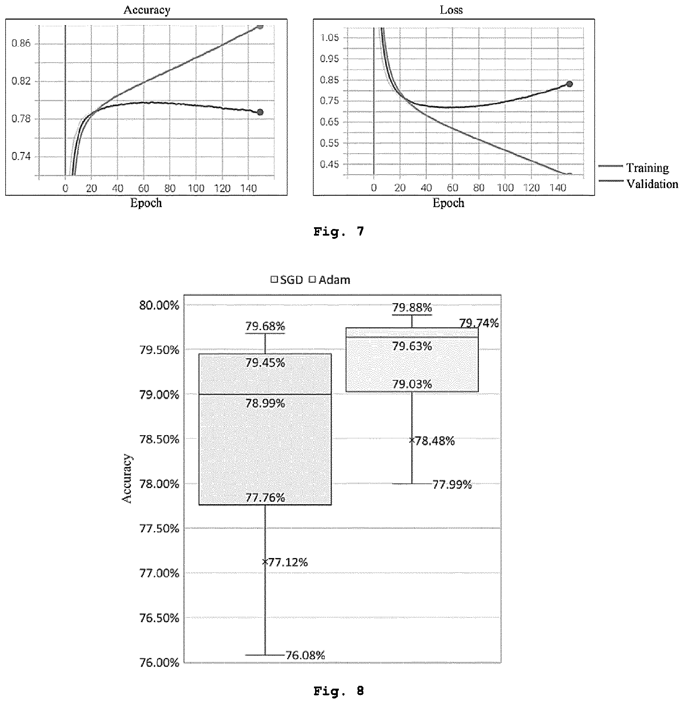

Based on the results observed on fine-tuning of the learning rates, we focus on adjusting the number of training epochs during this phase. We observe that the results clearly show a downward trend in loss and upward in accuracy with the learning rate of 1×10⁻⁶ on Adam optimizer (see Figure A1). This trend suggests that continuing to increase the value of epochs can potentially further improve the performance of the model. Although the best testing accuracy is obtained with a learning rate of 1×10⁻⁵, its learning curve doesn't show an obvious downward trend (see Figure A1). So, we choose the learning rate of 1×10⁻⁶ for further fine-tuning. We experiment by increasing the number of epochs from 10 to 150 and keeping the other hyperparameters the same. The training and validation curve is presented in Figure 6. The increasing trend of the loss on the validation dataset is a sign of overfitting. As presented in Figure 6, the loss curve shows the beginnings of this type of pattern after 41 epochs. This is where the model overfits the training dataset at the cost of worse performance on the validation dataset. These observations suggest that the best prediction model can be obtained after 41 epochs. The accuracy on the test dataset achieves a score of 79.96%. Now we turn to the model trained with the SGD optimizer. As the loss decreases most quickly on SGD is when setting the learning rate to 1×10⁻⁴ (see Figure A2). Similar to the experiment carried out on Adam, we use SGD as the optimizer and set the learning rate to 1×10⁻⁴. The results after 150 epochs are presented in Figure 7. we also observe that the runs show the beginnings of overfitting on the validation dataset after 67 epochs, and the best prediction model can be potentially obtained after 67 epochs. The accuracy on the test dataset achieves a score of 79.76%.

### Fine-Tuning on Batch Size

A small batch size can improve the performance, we further fine-tune our models by choosing different batch sizes within the set of {256, 128, 64, 32, 16}. Since the model trained with Adam optimizer and the learning rate of 1×10⁻⁶ is able to achieve better results than the other models after 150 epochs, the final tuning is carried out on this model. As observed in Figure 6, the validation accuracy does not change too much and begins to drop after 41 epochs, which means the model begins to converge. So, we choose the epoch of 50 on our final tuning within the batch size set (refer Appendix B Figure A3). The model trained with a batch size of 16 is able to surpass all the other models. The accuracy on the test dataset achieves a score of 80.57%. In summary, all the models trained during the fine-tuning process are evaluated in testing accuracy and the results are presented in Table 4.

**Table 4. Model results obtained during the fine-tuning process. All the models use the transformers-based architecture. The hyperparameters that are not listed in this table use the same settings presented in Table 3.**

| **Optimizer** | **Learning Rate** | **Epochs** | **Batch Size** | **Testing Accuracy** |
|---|---|---|---|---|
| Adam | 0.1 | 10 | 128 | 6.57% |
| Adam | 0.01 | 10 | 128 | 9.05% |
| Adam | 1 × 10⁻³ | 10 | 128 | 14.02% |
| Adam | 1 × 10⁻⁴ | 10 | 128 | 78.01% |
| Adam | 1 × 10⁻⁵ | 10 | 128 | 79.67% |
| Adam | 1 × 10⁻⁶ | 10 | 128 | 76.57% |
| Adam | 1 × 10⁻⁷ | 10 | 128 | 52.32% |
| | | | | |

| **Optimizer** | **Learning Rate** | **Epochs** | **Batch Size** | **Testing Accuracy** |
|---|---|---|---|---|
| SGD | 0.1 | 10 | 128 | 12.12% |
| SGD | 0.01 | 10 | 128 | 73.76% |
| SGD | 1 × 10⁻³ | 10 | 128 | 79.72% |
| SGD | 1 × 10⁻⁴ | 10 | 128 | 75.01% |
| SGD | 1 × 10⁻⁵ | 10 | 128 | 38.89% |
| SGD | 1 × 10⁻⁶ | 10 | 128 | 15.01% |
| SGD | 1 × 10⁻⁷ | 10 | 128 | 6.26% |
| Adam | 1 × 10⁻⁶ | 150 | 128 | 79.96%, |
| SGD | 1 × 10⁻⁴ | 150 | 128 | 79.76% |
| Adam | 1 × 10⁻⁶ | 50 | 16 | 80.57% |
| Adam | 1 × 10⁻⁶ | 50 | 32 | 80.19% |
| Adam | 1 × 10⁻⁶ | 50 | 64 | 80.20% |
| Adam | 1 × 10⁻⁶ | 50 | 128 | 80.25% |
| Adam | 1 × 10⁻⁶ | 50 | 256 | 80.08% |

### Results

We consider three deep neural network architectures in our experiments. We use these neural networks combined with different representations for words and texts. These built models and their respective testing accuracies results are presented in Table 5. Among them, the transformers-based model using BERT-base language model achieves a result of 78.15%. The CNN-based approach achieved an accuracy of 76.56%, and the RNN-based approach achieved an accuracy of 75.88%. The transformers-based approach is shown to be better than the one using the CNN-based or RNN-based approach. We then fine-tune the transformers-based models. A better model with a higher accuracy score is obtained after we fine-tune the hyperparameters on learning rate, optimizer, number of epochs, and batch size step by step. The best fine-tuning model achieves an accuracy of 80.57% on the testing set.

**Table 5. Results comparison among the three deep learning architectures.**

| **Deep Learning Architecture** | **Fine-Tuned Hyperparameters** | **Testing Accuracy** |
|---|---|---|
| CNN-based architecture | - | 76.56% |
| RNN-based architecture | - | 75.88% |
| Transformers-based architecture | - | 78.15% |
| | learning rate | 79.67% (Adam, Ir = 1 × 10⁻⁵) |
| | learning rate | 79.72% (SGD, Ir = 1 × 10⁻³) |
| | epochs | 79.96% (Adam, epochs = 150) |
| | epochs | 79.76% (SGD, epochs = 150) |
| | batch size | 80.57% |

We also compare the performance between the Adam and SGD optimizers using their validation accuracy distributions over the 150 epochs. In Figure 8, we present the results with a box plot using the data that both optimizers achieve with their best learning rate, where Adam has a learning rate of 1×10⁻⁶ and SGD has a rate of 1×10⁻⁴ The outliers that are smaller than the minimum for both optimizers are removed in the figure for a clear presentation. This does not affect the comparison between the distributions. We choose a learning rate of 1×10⁻⁶ for Adam and 1×10⁻⁴ for SGD because their learning curves show clear downward trends in losses and upward trends in accuracies. Also, they are able to achieve comparative performances compared to other learning rates (see Figures A1 and A2).

We observe that the model with Adam optimizer achieves better top accuracy on the validation dataset than the model with SGD. Besides, when comparing Figures 6 and 7, Adam has been found to attain the top accuracy performance with a smaller number of epochs necessary (epoch = 41), while SGD requires more to converge (epoch = 47). These findings suggest that: (i) The value of the learning rate and the optimizer choice can have a strong influence on each other, in other words, the performance of the built models highly depends on the combination of the learning rate and the optimizer; (ii) With the appropriate learning rate setting, Adam outperforms SGD not only in final generalization performance but also able to converge with a smaller number of epochs. The performances between the shallow machine learning techniques and neural networks are presented in Table 6.

**Table 6. Comparison between the neural network and shallow machine learning techniques on the SNS24 clinical triage classification task.**

| **Machine Learning Techniques** | **Architecture/Algorithm** | **Testing Accuracy** |
|---|---|---|
| Neural network | CNN-based architecture | 76.56% |
| | RNN-based architecture | 75.88% |
| | Transformers-based architecture | 80.57% |
| Shallow machine learning | Linear SVM | 77.96% |
| | RBF SVM | 76.97% |
| | Random Forest | 74.93% |
| | Multinomial NB | 66.26% |

### Analysis among Similar Clinical Symptoms

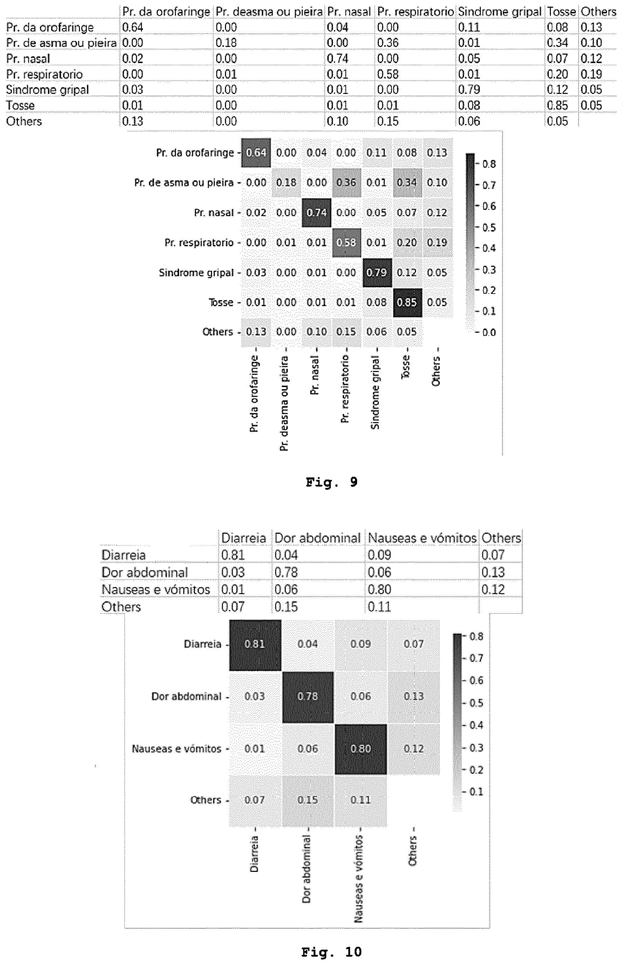

In this section, we performed an analysis of the trained neural network models when dealing with classes that have similar clinical symptoms. According to the call center, there is some degree of overlap between clinical pathways. Although this overlap is expected, it may make cause the clinical decision-making process to be more complex. Figures 9 and 10 presents two example groups that includes clinical pathways with similar symptoms. Figure 9 presents an example group that includes six clinical pathways with similar symptoms. They are "Pr. da orofaringe" (Oropharynx problem), "Pr. deasma or pieira" (Asthma or wheezing problem), "Pr. Nasal" (Nasal problem), "Pr. Respiratorio" (Respiratory problem), "Sindrome gripal" (Flu syndrome), and "Tosse" (Cough). Using the pre-trained neural network model, we built the heatmap for these clinical pathways. Taking Oropharynx problem for example, 64% of the cases are rightly classified,
4% are wrongly classified as Nasal problem, 11% as Flu syndrome, 8% as Cough, and 13% as the other clinical pathways. On the other way around, 3% Flu syndrome cases are wrongly classified as Oropharynx problem, and 12 % as Cough.

These observations suggest that Flu syndrome cases are more likely to be classified as Cough than Oropharynx problem. But on the other way, the Oropharynx problem case is more likely to be classified as Flu syndrome than Cough. Furthermore, we find that 1% of the Cough cases are wrongly classified as Oropharynx problem and 8% as Flu syndrome, which means that Cough cases are more likely to be classified as Flu syndrome. Joining these above observations, we can deduce that it is harder to distinguish between Cough and Flu syndrome cases, and in practical application, the nurses should pay more attention to identifying these two types of clinical pathways.

Figure 10 presents an example group that includes three clinical pathways with similar symptoms. They are "Diarreia" (Diarrhea), "Dor abdominal" (Abdominal pain), and "Náuseas e vômitos" (Nausea and vomiting).

As can be observed, an average of 80% cases in this group can be rightly classified with the built neural network model. This means that the built model can provide useful suggestions to the nurses when distinguish the clinical pathways that fall into this group. We also find that for Abdominal pain, 6% are wrongly classified as Nausea and vomiting, and 3% as Diarrhea. This means that Abdominal pain is more likely to be classified as Nausea and vomiting. Similarly, when observing Nausea and vomiting, 6% are classified as Abdominal pain and 1% as Diarrhea. These findings suggest that Abdominal pain and Nausea and vomiting cases are harder to be distinguished in this group. These results provide valuable analysis for the Health Calls center, for example, helping to do deep analysis between similar symptoms.

## Claims

1. Text classification method to aid on clinical referral of patients based on the selection of a clinical pathway, said method comprising
collecting personal and clinical information about a patient; inserting the personal and clinical information in text format in an application form;
obtaining through the application form a text result based on the inserted personal and clinical information that identifies the clinical pathways most probable for the patient symptoms.

2. Text classification method according to the previous claim, wherein the application form is instanced through a web application service supported by a database.

3. Text classification method according to any of the previous claims, wherein the web application service comprises at least one of three deep learning based classification architectures, particularly a convolutional neural network or a recurrent neural network or transformers-based architecture.

4. Text classification method according to any of the previous claims, wherein the web application service comprises the steps of
recording the personal and clinical information of a patient; pre-processing the personal and clinical information;
applying the personal and clinical information to the at least one of three deep learning based classification architectures; obtaining a prediction of the most probable clinical pathways.

5. Text classification method according to any of the previous claims, wherein the web application service comprises
an input layer where the personal and clinical information of a patient comprising contact reason will be inserted;
at least one hidden layer composed at least of embeddings and pre-trained word embeddings or language models; and
an output layer composed of the prediction.

6. Text classification method according to any of the previous claims, wherein the web application service comprises the steps of Fine-Tuning of the transformers-based architecture results.
